# EUROPEAN PATENT APPLICATION

(11) **EP 1 875 904 A1**
(43) Date of publication of application: **09.01.2008**
(21) Application number: 07017518.7
(22) Date of filing: 29.08.2003
(51) Int. Cl.: A61K 31/19, A61K 31/20, A61P 3/06, A61P 3/10, A61P 19/02, A61P 29/00, A61P 35/00, A61K 49/00, G01N 33/50

(54) **Methods**

(30) Priority: 29.08.2002 GB 0220045
(62) Divisional of application: 03750887.6
(71) Applicant: CXR BIOSCIENCES LIMITED, Dundee DD1 5JJ (GB)
(72) Inventor: Elcombe, Clifford Roy, Dundee, DD 5JJ (GB); Wolf, Charles Roland, Dundee, DD 5JJ (GB)
(74) Representative: Pilkington, Stephanie Joan

(57) **Abstract**

Use of a compound selected from perfluorohexanoic acid, perfluoropentanoic acid, perfluorobutanoic acid or perfluoropropionic acid, or a salt or an ester of any thereof, or perfluorooctane, in the manufacture of a medicament for treating a patient in need of modulation of one or both of plasma insulin and plasma glucose, or for treating a patient who has diabetes or who is at risk of developing diabetes.

## Description

This invention relates to the medical use of compounds, and to methods of identifying further useful compounds, particularly in the treatment of diabetes, obesity, hyperlipidaemia, hypercholesterolaemia, atherosclerosis, cancer and inflammation, or other conditions where alterations in lipid or eicosanoid status may be desirable.

Perfluorooctanoic acid (PFOA) and other perfluorinated fatty acids or fluoroalkyl molecules are synthetic molecules used in industrial applications, principally as surfactants. The effects of such compounds on laboratory animals and cells has been studied, as have the effects of occupational exposure in humans (see, for example, Gilliland & Mandel (1993) J Occup Med 35(9), 950-954; Kees et al (1992) J Med Chem 35, 944-953). US 4,624,851 suggests treatment of symptoms of cancer using fluorine containing acids; no experimental data is presented.

We have surprisingly found that particular such compounds may have beneficial effects. We have found that particular such compounds may be useful in treatment of diabetes, obesity, hyperlipidaemia, hypercholesterolaemia, atherosclerosis, cancer and inflammation, or other conditions where alterations in lipid or eicosanoid status may be desirable. We have further found that particular compounds or combinations of compounds may be particularly useful in treatment of one or more of these conditions.

A first aspect of the invention provides a method of treatment of a patient in need of modulation (preferably reduction) of body mass or modulation (preferably prevention or reduction) of increase in body mass, and/or in need of modulation (preferably reduction) of plasma insulin, plasma glucose, plasma triglycerides and/or plasma cholesterol, comprising administering to the patient an effective amount of a perfluoroctanoic acid or a salt (preferably other than ammonium salt, preferably other than 75% straight chain ammonium salt) or an ester thereof; perfluorosuberic acid, perfluoroheptanoic acid, perfluorohexanoic acid, perfluoropentanoic acid, perfluorobutanoic acid or perfluoropropionic acid or a salt or an ester any thereof; or perfluoroctane.

A further aspect of the invention provides a method of treatment of a patient in need of an antitumour agent or an antiinflammatory agent, or in need of modulation in lipid or eicosanoid status, comprising administering to the patient an effective amount of a perfluoroctanoic acid or a salt (preferably other than ammonium salt, preferably other than 75% straight chain ammonium salt) or an ester thereof; perfluorosuberic acid, perfluoroheptanoic acid, perfluorohexanoic acid, perfluoropentanoic acid, perfluorobutanoic acid or perfluoropropionic acid or a salt or an ester any thereof; or perfluoroctane. Such compounds, particularly perfluorooctanoic acid or perfluoroheptanoic acid) are considered to be effective as an antitumour agent or an antiinflammatory agent or in modulating lipid or eicosanoid status (ie type and concentration of lipid or eicosanoid, either systemically or in a particular locus or tissue).

The patient may be a patient with or at risk of excessive inflammation, for example with or at risk of arthritis, or a patient with or at risk of developing a tumour. The compound may reduce the development, growth or metastasis of a tumour.

The compound may be useful in treating any condition or disorder in which the patient has or is at risk of excessive inflammation. The patient may have an allergic or autoimmune disease. The patient may have, for example, psoriasis, inflammatory bowel disease, asthma or rheumatism.

A further aspect of the invention provides a method of treatment of a patient who is overweight or obese and/or has diabetes, hyperlipidaemia, atherosclerosis, coronary heart disease, stroke, obstructive sleep apnoea, arthritis (for example osteoarthritis) and/or reduced fertility, or is at risk of developing such a condition, comprising administering to the patient an effective amount of a perfluoroctanoic acid or a salt (preferably other than ammonium salt, preferably other than 75% straight chain ammonium salt) or an ester thereof; perfluorosuberic acid, perfluoroheptanoic acid, perfluorohexanoic acid, perfluoropentanoic acid, perfluorobutanoic acid or perfluoropropionic acid or a salt or an ester any thereof; or perfluoroctane.

A further aspect of the invention provides a method of treatment of a patient in need of modulation of PPAR (for example PPARα, δ or γ) activity, comprising administering to the patient an effective amount of a perfluoroctanoic acid or a salt (preferably other than ammonium salt, preferably other than 75% straight chain ammonium salt) or an ester thereof; perfluorosuberic acid, perfluoroheptanoic acid, perfluorohexanoic acid, perfluoropentanoic acid, perfluorobutanoic acid or perfluoropropionic acid or a salt or an ester any thereof; or perfluoroctane. Such a compound may be a PPAR agonist or a PPAR antagonist; it may be an agonist for one PPAR and an antagonist for a different PPAR. For example, perfluorosuberic acid and salts and esters thereof are considered to be PPARγ agonists but not PPARα agonists; they may be PPARα antagonists. Perfluoroctanoic acid, perfluoroheptanoic acid, perfluorohexanoic acid, perfluoropentanoic, perfluorobutanoic acid and perfluoropropionic acid and salts and esters any thereof are considered to be PPARα and PPARγ agonists. Perfluorooctanoic acid, perfluorosuberic acid, perfluoroheptanoic acid, perfluorohexanoic acid, perfluoropentanoic acid, perfluorobutanoic acid and perfluoropropionic acid (but possibly not salts and esters any thereof) are considered to be PPARδ antagonists. Preferably the patient is in need of an increase in PPARα or PPARγ activity and the compound is a PPARα or PPARγ agonist. Alternatively, the patient may be in need of a decrease in PPARα or PPARγ activity and the compound may be a PPARα or PPARγ antagonist. In a still further alternative, the patient may be in need of a decrease in PPARδ activity and the compound may be a PPARδ antagonist. PPARδ may have opposing effects to PPARα or PPARγ (see, for example, WO01/07066).

A further aspect of the invention provides a method of treatment of a patient in need of modulation of lipid or eicosanoid status or function, for example in need of modulation of the activity of a lipid metabolising or binding entity (including a lipid metabolising enzyme and a lipid binding polypeptide, for example a lipid transporting polypeptide), for example cycloxygenase (for example cyclooxygenase I or cyclooxygenase II) activity or phospholipase A (for example phospholipase A2) or lipoxygenase, comprising administering to the patient an effective amount of a perfluoroctanoic acid or a salt (preferably other than ammonium salt, preferably other than 75% straight chain ammonium salt) or an ester thereof; perfluorosuberic acid, perfluoroheptanoic acid, perfluorohexanoic acid, perfluoropentanoic acid, perfluorobutanoic acid or perfluoropropionic acid or a salt or an ester any thereof; or perfluoroctane. Preferably the patient is in need of a decrease in a lipid metabolising or binding activity, for example cycloxygenase (for example cyclooxygenase I or cyclooxygenase II) activity or phospholipase A or lipoxygenase and the compound is an inhibitor of such activity. For example, inappropriate lipoxygenase activity may be involved in inflammation, hypersensitivity, asthma and some vascular diseases; thus a decrease in a lipoxygenase activity may be useful in such a condition.

Alternatively, the patient may be in need of an increase in such activity and the compound may be an activator of such activity.

Further preferences in relation to the patient and compound are indicated below.

The compounds may exhibit tautomerism. All tautomeric forms and mixtures thereof are included within the scope of the invention.

The compounds may also contain one or more asymmetric carbon atoms and may therefore exhibit optical and/or diastereoisomerism. Diastereoisomers may be separated using conventional techniques, e.g. chromatography or fractional crystallisation. The various stereoisomers may be isolated by separation of a racemic or other mixture of the compounds using conventional, e.g. fractional crystallisation or HPLC, techniques. Alternatively the desired optical isomers may be made by reaction of the appropriate optically active starting materials under conditions which will not cause racemisation or epimerisation, or by derivatisation, for example with a homochiral acid followed by separation of the diastereomeric esters by conventional means (e.g. HPLC, chromatography over silica). All stereoisomers are included within the scope of the invention.

In an embodiment the perfluoroctane, perfluorooctanoic acid and perfluoroheptanoic acid are straight chain but this is not considered to be essential. For example, the perfluoroctane, perfluorooctanoic acid and perfluoroheptanoic acid may be at least 50%, 60, 70, 80, 90 or 95% straight chain, for example 75% straight chain or 100% straight chain.

By the term "ester" is included those formed with an alcohol of formula R¹OH, wherein R¹ represents aryl or alkyl; and those formed with a thiol of formula R¹SH, wherein R¹ is as hereinbefore defined (ie a thioester). It is preferred that the ester is not a thioester. In embodiments R¹ represents C₁₋₆ alkyl, for example C₁ alkyl (eg methyl).

By the term "salt" is included, for example, those formed with a nitrogen-containing base such as ammonia, an alkylamine, a dialkylamine, a trialkylamine and pyridine or alkali or alkaline earth metal salts (e.g. Na, K, Cs, Mg or Ca salts).

Preferred compounds include those that are pharmaceutically acceptable.

The term "aryl", when used herein, includes C₆₋₁₀ aryl groups such as phenyl, naphthyl and the like. Aryl groups may be substituted by one or more substituents including -OH, cyano, halo, nitro, amino, alkyl and alkoxy. When substituted, aryl groups are preferably substituted by between one and three substituents.

The term alkyl, when used herein, refers to alkyl groups of 1 to 16, preferably 1 to 10 (e.g. 1 to 6) carbon atoms.

The term alkoxy, when used herein, refers to alkoxy groups of 1 to 16, preferably 1 to 10 (e.g. 1 to 6) carbon atoms.

Alkyl and alkoxy groups as defined herein may be straight-chain or, when there is a sufficient number (i.e. a minimum of three) of carbon atoms, be branched-chain and/or cyclic and/or heterocyclic. Further, when there is a sufficient number (i.e. a minimum of four) of carbon atoms, such alkyl and alkoxy groups may also be part cyclic/acyclic. Such alkyl and alkoxy groups may also be saturated or, when there is a sufficient number (i.e. a minimum of two) of carbon atoms, be unsaturated and/or interrupted by one or more oxygen and/or sulfur atoms. Alkyl and alkoxy groups may also be substituted by one or more halo, and especially fluoro, atoms.

The term "halo", when used herein, includes fluoro, chloro, bromo and iodo.

The terms alkylamine, dialkylamine and trialkylamine, when used herein, refer to amines bearing one, two or three alkyl groups as defined herein, respectively.

The term alkylene, when used herein, refers to alkylene groups of 1 to 20, preferably 2 to 17 (e.g. 6 to 12) carbon atoms. Alkylene groups may be straight-chain or, when there is a sufficient number (i.e. a minimum of two or three, as appropriate) of carbon atoms, be branched-chain and/or cyclic and/or heterocyclic. Further, when there is a sufficient number (i.e. a minimum of four) of carbon atoms, such alkylene groups may also be part cyclic/acyclic. Such alkylene chains may also be saturated or, when there is a sufficient number (i.e. a minimum of two) of carbon atoms, be unsaturated and/or interrupted by one or more oxygen and/or sulfur atoms.

Particularly preferred compounds may be or comprise a member of the following group:
Perfluoroheptanoic acid; perfluorohexanoic acid; perfluorooctanoic acid; perfluorosuberic acid; perfluoropentanoic acid; perfluorobutanoic acid; perfluoropropanoic acid; methyl perfluoroheptanoate; methyl perfluorohexanoate; methyl perfluorooctanoate; methyl perfluoropentanoate; methyl perfluorbutanoate; methyl perfluoropropionate; dimethyl perfluorosuberate. Compounds may be obtained from any suitable supplier, for example 3M, Sigma, DuPont, Miteni or Dyneon.

The chemical formula for APFO is CF₃(CF₂)₆COO⁻ NH₄⁺ (octanoic acid, pentadecafluoro-, ammonium salt; C-8, FC-143; CAS Registry No 3825-26-1). It may be obtained from DuPont (DuPont Chemical Solutions Enterprise, DuPont-Strassel, D-61343 Bad Homburg, Germany). Common contaminants of APFO include ammonium perfluoroheptanoate (CAS 6130-43-4), ammonium perfluorohexanoate (CAS 68259-11-0), ammonium perfluoropentanoate (CAS 21615-47-4), and branched chain homologs that are generically known as ammonium perfluoroisooctanoate, ammonium perfluoroisoheptanoate, ammonium perfluoroisohexanoate and ammonium perfluoroisopentanoate. Whilst it is considered that the effects observed in Example 1 using an APFO preparation arise from the administration of APFO itself, it will be appreciated that one or more contaminants, for example one or more of the possible contaminants listed above, may contribute to the effects observed. 100% straight chain APFO may be more potent than, for example, 75% straight chain APFO, as discussed in Example 2.

It is preferred that the compound is metabolically stable; for example it is preferred that the compound has a similar rate of metabolism to perfluorooctanoic acid. The compound may be considered to be a lipid mimetic which may be metabolically stable. Metabolism of an ester or salt to the free acid is acceptable.

A further aspect of the invention provides the use of a perfluoroctanoic acid or a salt (preferably other than ammonium salt, preferably other than 75% straight chain ammonium salt) or an ester thereof; perflurosuberic acid, perfluoroheptanoic acid, perfluorohexanoic acid, perfluoropentanoic acid, perfluorobutanoic acid or perfluoropropionic acid or a salt or an ester any thereof; or perfluoroctane in the manufacture of a medicament for the treatment of a patient in need of modulation (preferably reduction) of body mass or modulation (preferably prevention or reduction) of increase in body mass, and/or in need of modulation (preferably reduction) of plasma insulin, plasma glucose, plasma triglycerides and/or plasma cholesterol. The patient may (for example in relation to a decrease in the above-listed parameters) be overweight or obese and/or have diabetes, hyperlipidaemia and/or atherosclerosis, or be at risk of developing such a condition. The risk may arise from genetic factors, age, or environmental factors, such as diet.

The patient may have other condition(s) associated with obesity, for example coronary heart disease, stroke, obstructive sleep apnoea, arthritis (for example osteoarthritis) or reduced fertility.

Accordingly, a further aspect of the invention provides the use of a perfluoroctanoic acid or a salt (preferably other than ammonium salt, preferably other than 75% straight chain ammonium salt) or an ester thereof; perflurosuberic acid, perfluoroheptanoic acid, perfluorohexanoic acid, perfluoropentanoic acid, perfluorobutanoic acid or perfluoropropionic acid or a salt or an ester any thereof; or perfluoroctane in the manufacture of a medicament for the treatment of a patient who is overweight or obese and/or has diabetes, hyperlipidaemia, atherosclerosis, coronary heart disease, stroke, obstructive sleep apnoea, arthritis (for example osteoarthritis) and/or reduced fertility, or is at risk of developing such a condition.

A further aspect of the invention provides the use of a perfluoroctanoic acid or a salt (preferably other than ammonium salt, preferably other than 75% straight chain ammonium salt) or an ester thereof; perflurosuberic acid, perfluoroheptanoic acid, perfluorohexanoic acid, perfluoropentanoic acid, perfluorobutanoic acid or perfluoropropionic acid or a salt or an ester any thereof; or perfluoroctane in the manufacture of a medicament for the treatment of a patient in need an antitumour agent or an antiinflammatory agent or of modulation of lipid or eicosanoid status. Preferences in relation to such a patient are noted above.

As is well known to those skilled in the art, obesity may be described as a state of excessive accumulation of body fat. Obesity may be determined by determining the body mass index (BMI) for a patient, and/or by measuring subcutaneous fat deposits in the arm using a "pinch test". The BMI is defined as weight (in kilograms) divided by the square of the height in metres. A BMI of 25-30 is considered as overweight and more than 30 as obese. Preferably, treatment leads to lowering of the BMI to less than about 29 to 31, or to a point at which health risks from being overweight are no longer significant.

It will be appreciated that the treatment of the invention may be used in combination with other treatments for the relevant condition. For example in relation to obesity, the patient may follow a calorie-restricted diet and/or follow a program of physical exercise.

The medicament may comprise more than one (e.g. two) of the indicated compounds. The medicament may comprise a prodrug, for example a molecule which is converted to a molecule with the required biological activity following administration of the medicament to the patient.

The compound may be particularly useful in the treatment of patients with diabetes. For example, the compound may be useful in treating type II diabetes. The compound may be, for example, perfluorooctanoic acid or a salt (preferably not the ammonium salt, preferably other than 75% straight chain ammonium salt) or ester (for example methyl ester) thereof, perfluoroheptanoic acid or a salt or ester thereof, perfluorohexanoic acid or a salt or ester thereof, perfluoropentanoic acid or a salt or ester thereof, or perfluorooctane. In type I diabetes, the compounds may be useful as an insulin sensitiser and may therefore allow the dose of insulin administered to be reduced, thereby lowering costs and potentially reducing side effects of insulin administration. Existing anti-diabetic agents, for example the thiazolidinedione class of agents, may have the undesirable effect of stimulating weight gain. The present compounds (particularly perfluorooctanoic acid and methyl perfluorooctanoate) are considered to have the desirable effect of preventing weight gain as well as being useful as anti-diabetic agents.

Compounds of the invention may be useful in the concomitant treatment of a number of abnormalities, for example diabetes, obesity and hyperlipidaemia. Thus, it may be possible to treat a patient with these conditions (which may often occur together) with a single compound or preparation. This may have benefits, for example in relation to patient compliance, the avoidance of drug interactions, ease of formulation and marketing.

It is preferred that the patient is mammalian, most preferably human or, less preferably a domesticated animal, for example an animal kept as a pet or in agriculture, for example horse, cow, cat or dog.

It is preferred that the compound is a compound wherein the plasma insulin levels are modulated (preferably reduced) in a mammal following administration of the compound to the mammal, relative to either preadministration levels or a control mammal which has not been administered the compound. It is particularly preferred that plasma insulin levels are modulated (for example reduced) (for example relative to a control animal) in a male Fischer 344 rat following administration of the compound to the rat, as described in Example 1. It is sufficient for a reduction to be found at any time following first administration of the compound to the mammal (preferably rat), but it is preferred that such reduction is found (ie appears or is still present) at least seven days after first administration of the compound. It is preferred that a reduction of insulin levels of at least 1, 2, 5, 10, 15, 20, 25, 30, 35, 40, 45, 50, 55, 60, 65, 70, 75 or 80% is achieved.

It will be appreciated that the comparative measurements are made on animals at substantially the same stage of feeding, ie at substantially the same time of day or at substantially the same time following ingestion of food.

When the mammal is a rat, it is preferred that plasma insulin levels are modulated (preferably reduced) following administration of the compound at a level of between 30 and 5000 or 3000ppm of the diet, preferably between about 50 and 500ppm, still more preferably about 300ppm. It is preferred that the test is conducted using the methods and conditions described in Example 1. It is preferred that the change in insulin level is not accompanied by any adverse clinical symptoms or change in behaviour/activity of the mammal. Thus, the animals may be observed in relation to standard clinical chemistry analyses, blood pressure and/or dizziness. Thus, it is preferred that insulin levels are modulated (preferably reduced) following administration of an amount of the compound that does not produce a significant adverse effect on the animal.

Tests may be performed on more than one animal for a compound or given dose of a compound, as known to those skilled in the art.

Alternatively or in addition, it is preferred that plasma cholesterol, glucose and/or trigylceride levels are modulated (preferably reduced), in a mammal following administration of the compound to the mammal, relative to either preadministration levels or a control mammal which has not been administered the compound. Preferences indicated above in relation to modulation (for example reduction) of insulin levels apply similarly in relation to modulation (for example reduction) of plasma cholesterol, glucose and trigylceride levels. Alternatively or in addition, eicosanoid status (ie type or concentration) may be modulated (preferably reduced) in a mammal (or in a particular locus or tissue) following administration of the compound to the mammal, relative to either preadministration levels or a control mammal which has not been administered the compound.

Alternatively or in addition, it is preferred that bodyweight or bodyweight gain is modulated (preferably reduced) in a mammal following administration of the compound to the mammal, relative to either preadministration levels (for bodyweight) or a control mammal (for bodyweight or bodyweight gain) which has not been administered the compound. Preferences indicated above in relation to modulation (for example reduction) of insulin levels apply similarly in relation to modulation (for example reductions) in bodyweight or bodyweight gain.

Food consumption (expressed as weight of food consumed per unit bodyweight) may be increased following administration of the compound to the mammal, relative to either preadministration levels or a control mammal which has not been administered the compound. The increase may not be seen immediately after commencing administration of the compound; an initial decrease may be seen, which may be followed by an increase.

Whilst not wishing to be bound by theory, it is considered that a compound as defined above may bind to a peroxisome proliferator activated receptor (PPAR), for example PPARα, PPARδ or PPARγ (Kliewer et al (1994) PNAS, 91, 7355-7359; reviewed in Gelman et al (1999) Cell Mol Life Sci 55, 932-943; Kersten et al (2000) Nature 405, 421-424 and Issemann & Green (1990) Nature 347, 645-650) and may be a PPAR agonist or antagonist. It is preferred that the compound binds to a peroxisome proliferator activated receptor (PPAR), for example PPARα, PPARδ or PPARγ. It is further preferred that the compound is a PPARα, PPARδ or PPARγ modulator, for example a PPARα, PPARδ or PPARγ antagonist or agonist or partial agonist (which can be defined as a compound that is unable to induce maximal activation of the receptor population regardless of the amount of compound applied). It is particularly preferred that the compound is a PPARα or PPARγ agonist or partial agonist or a PPARδ antagonist. Any suitable method may be used for determining whether a compound binds to and/or is a modulator, for example an agonist, partial agonist or antagonist of a PPAR.

Also whilst not wishing to be bound by theory, a compound as defined above may bind to a lipid metabolising or binding entity, for example a cycloxygenase, for example COXI or COXII, or phospholipase A, for example Phospholipase A2, or lipoxygenase and may be a modulator, for example an activator or inhibitor, of such an entity's activity (including degree of activation). It is preferred that the compound binds to a lipid metabolising enzyme, for example a cycloxygenase, for example COXI or COXII. It is further preferred that the compound is a modulator of the activity of a lipid metabolising enzyme, for example a cycloxygenase, for example COXI or COXII. Any suitable method may be used for determining whether a compound binds to and/or is a modulator, for example an activator or inhibitor of a lipid binding or metabolising entity.

A further aspect of the invention provides the use of a perfluoroctanoic acid or a salt (preferably other than ammonium salt, preferably other than 75% straight chain ammonium salt) or an ester thereof; perflurosuberic acid, perfluoroheptanoic acid, perfluorohexanoic acid, perfluoropentanoic acid, perfluorobutanoic acid or perfluoropropionic acid or a salt or an ester any thereof; or perfluoroctane in the manufacture of a medicament for treating a patient in need of modulation of PPAR (for example PPARα, PPARδ (also known as β) or PPARγ) activity. Preferably the patient is in need of an increase in PPAR (preferably PPARα and/or PPARγ) activity and the compound is a PPAR (for example a PPARα or γ) agonist, as discussed above. Alternatively, the patient is in need of a decrease in PPAR (preferably PPARδ) activity and the compound is a PPAR (for example a PPARδ) antagonist, as discussed above.

A further aspect of the invention provides the use of a perfluoroctanoic acid or a salt (preferably other than ammonium salt, preferably other than 75% straight chain ammonium salt) or an ester thereof; perflurosuberic acid, perfluoroheptanoic acid, perfluorohexanoic acid, perfluoropentanoic acid, perfluorobutanoic acid or perfluoropropionic acid or a salt or an ester any thereof; or perfluoroctane in the manufacture of a medicament for treating a patient in need of modulation of a lipid metabolising entity activity, for example cycloxygenase (for example cyclooxygenase I or cyclooxygenase II) activity or phospholipase A (for example phospholipase A2) activity or lipoxygenase activity.

A further aspect of the invention provides a screening method for identifying a drug-like compound or lead compound for the development of a drug-like compound in which (1) a mammal is exposed to a perfluoroctanoic acid or a salt (preferably other than ammonium salt, preferably other than 75% straight chain ammonium salt) or an ester thereof; perflurosuberic acid, perfluoroheptanoic acid, perfluorohexanoic acid, perfluoropentanoic acid, perfluorobutanoic acid or perfluoropropionic acid or a salt or an ester any thereof; or perfluoroctane (2) the plasma insulin, glucose, cholesterol or triglyceride level of the mammal is measured, and/or bodyweight of the mammal is measured, and/or lipid or eicosanoid status (ie type and level of at least one lipid or eicosanoid) or function (for example assessed by degree of responsiveness to the mammal to a lipid or eicosanoid) of the mammal is measured.

The method preferably comprises the step of selecting a compound on exposure to which the plasma insulin, glucose, cholesterol, and/or triglyceride level of the mammal is changed, preferably reduced, and/or bodyweight or bodyweight increase is changed, preferably reduced. Preferences for this aspect of the invention include those indicated above in relation to investigating effects on insulin, cholesterol, glucose or triglyceride levels, or on bodyweight. For example, it is preferred that the mammal is a rodent, for example a rat or a mouse, or other laboratory animal such as a dog.

A further aspect of the invention provides a screening method for identifying a drug-like compound or lead compound for the development of a drug-like compound in which (1) a mammal is exposed to a a perfluoroctanoic acid or a salt (preferably other than ammonium salt, preferably other than 75% straight chain ammonium salt) or an ester thereof; perflurosuberic acid, perfluoroheptanoic acid, perfluorohexanoic acid, perfluoropentanoic acid, perfluorobutanoic acid or perfluoropropionic acid or a salt or an ester any thereof; or perfluoroctane (2) the plasma insulin, glucose, cholesterol or triglyceride level of the mammal is measured, and/or bodyweight of the mammal is measured, and/or lipid or eicosanoid status (ie type and level of at least one lipid or eicosanoid) or function (for example assessed by degree of responsiveness to the mammal to a lipid or eicosanoid) of the mammal is measured.

A further aspect of the invention provides a screening method for identifying a drug-like compound or lead compound for the development of a drug-like compound in which (1) a perfluoroctanoic acid or a salt (preferably other than ammonium salt, preferably other than 75% straight chain ammonium salt) or an ester thereof; perflurosuberic acid, perfluoroheptanoic acid, perfluorohexanoic acid, perfluoropentanoic acid, perfluorobutanoic acid or perfluoropropionic acid or a salt or an ester any thereof; or perfluoroctaneis exposed to a PPAR polypeptide (2) the binding of the compound to the PPAR polypeptide is measured or the change in the activity of the PPAR polypeptide is measured. Suitable methods by which binding of the compound to the PPAR polypeptide or effect on activity of the PPAR polypeptide may be measured are described, for example, in Example 1 or in US 6,028,109. The method may comprise the step of selecting a compound that binds to the PPAR polypeptide and/or changes its activity, for example nucleic acid binding activity and/or transcription factor activity. It is preferred that the selected compound increases PPARα or PPARγ activity ie acts as a PPARα or PPARγ agonist, or decreases PPARδ activity, ie acts as a PPARδ antagonist.

A further aspect of the invention provides a screening method for identifying a drug-like compound or lead compound for the development of a drug-like compound in which (1) a perfluoroctanoic acid or a salt (preferably other than ammonium salt, preferably other than 75% straight chain ammonium salt) or an ester thereof; perflurosuberic acid, perfluoroheptanoic acid, perfluorohexanoic acid, perfluoropentanoic acid, perfluorobutanoic acid or perfluoropropionic acid or a salt or an ester any thereof; or perfluoroctane is exposed to a lipid metabolising or binding entity, for example cycloxygenase (for example cyclooxygenase I or cyclooxygenase II) or phospholipase A (for example phospholipase A2) (2) the binding of the compound to the lipid metabolising or binding entity is measured or the change in the activity of the lipid metabolising or binding entity is measured. Suitable methods by which binding of the compound to the lipid metabolising or binding entity or effect on activity of the lipid metabolising or binding entity may be measured will be well known to those skilled in the art. Methods similar to those described in, for example, US 6,028,109, may be suitable, as noted above. The method may comprise the step of selecting a compound that binds to the lipid metabolising or binding entity and/or changes its activity, for example production of arachidonic acid from appropriate phospholipid (phospholipase A) or production of prostaglandin from arachidonic acid (cyclooxygenase). It is preferred that the selected compound decreases the enzymic or binding activity-ie acts as an inhibitor of the enzyme or binding entity.

A screening method of the invention may involve comparing the effect achieved using the test compound with that achieved using APFO or PFOA or other compound with desirable properties, as indicated above. A screening method of the invention may involve determining whether the test compound is able to compete with APFO or PFOA or other compound with desirable properties, as indicated above, for example whether it competes with APFO or PFOA for binding to a PPAR polypeptide, for example PPARα, or other lipid metabolising or binding entity, for example COXI, COXII or phospholipase A2.

Useful screening methods (for example in which the effect of the test compound is compared with that of APFO or PFOA or other compound with desirable properties, as indicated above) also include lipid displacement assays, cell (for example adipocyte) differentiation assays, or other phenotypic assays, insulin sensitisation assays, antiniflammatory screen, or investigation of effects on eicosanoid biosynthesis. The compound may be tested in animal models useful in investigating conditions of interest as noted above, such as obesity, diabetes, hyperlipidaemia or carcinogenesis. Such models include obese (ob/ob) or diabetic (db/db) mice, APC/min mice, BB rat or human tumour xenograft models, as known to those skilled in the art.

A further aspect of the invention provides a screening method for identifying a drug-like compound or lead compound for the development of a drug-like compound in which (1) a cell is exposed to a perfluoroctanoic acid or a salt (preferably other than ammonium salt, preferably other than 75% straight chain ammonium salt) or an ester thereof; perflurosuberic acid, perfluoroheptanoic acid, perfluorohexanoic acid, perfluoropentanoic acid, perfluorobutanoic acid or perfluoropropionic acid or a salt or an ester any thereof; or perfluoroctane (2) the phenotype (for example differentiation) and/or eicosanoid biosynthesis of the cell is measured. The method preferably comprises the step of selecting a compound on exposure to which the phenotype, for example differentiation, of the cell is changed, and/or eicosanoid biosynthesis of the cell is changed, preferably reduced.

The screening methods may be useful in identifying a drug-like compound or lead compound for the development of a drug-like compound for treating diabetes, obesity, hypercholesterolaemia and/or hyperlipidaemia.

The methods may further comprise the step of determining whether the compound is toxic or carcinogenic, for example at a concentration sufficient to elicit a change in bodyweight or bodyweight gain, plasma insulin, glucose, cholesterol and/or triglyceride levels. Such methods will be well known to those skilled in the art.

It will be appreciated that the compound may preferably be tested in more than of the screening methods of the invention. For example, a compound may be tested for its effect on a PPAR polypeptide, and for its effect on a mammal to which it is administered. The toxicity or carcinogenicity of the compound may also be determined.

The term "drug-like compound" is well known to those skilled in the art, and may include the meaning of a compound that has characteristics that may make it suitable for use in medicine, for example as the active ingredient in a medicament. Thus, for example, a drug-like compound may be a molecule that may be synthesised by the techniques of organic chemistry, less preferably by techniques of molecular biology or biochemistry, and is preferably a small molecule, which may be of less than 5000 daltons molecular weight. A drug-like compound may additionally exhibit features of selective interaction with a particular protein or proteins and be bioavailable and/or able to penetrate cellular membranes, but it will be appreciated that these features are not essential.

The term "lead compound" is similarly well known to those skilled in the art, and may include the meaning that the compound, whilst not itself suitable for use as a drug (for example because it is only weakly potent against its intended target, non-selective in its action, unstable, difficult to synthesise or has poor bioavailability) may provide a starting-point for the design of other compounds that may have more desirable characteristics.

These "lead" compounds may then be developed further, for example by molecular modelling/and or experiments to determine a structure activity relationship, in order to develop more efficacious compounds, for example by improving potency, selectivity/specificity and pharmacokinetic properties.

The methods may be performed *in vitro,* either in intact cells or tissues (for example liver cells or adipocytes), with broken cell or tissue preparations or at least partially purified components. Alternatively, they may be performed *in vivo*. The cells tissues or organisms in/on which the use or methods are performed may be transgenic. In particular they may be transgenic for a PPAR polypeptide or lipid metabolising or binding entity.

It will be appreciated that the polynucleotide encoding the PPAR (for example PPARα, β or γ) or lipid metabolising or binding entity may be mutated in order to encode a variant of the PPAR, for example by insertion, deletion, substitution, truncation or fusion, as known to those skilled in the art. It is preferred that the PPAR or lipid metabolising or binding entity is not mutated in a way that may materially affect its biological behaviour, for example its nucleic acid binding or transcription factor activity or lipid metabolising or binding activity, as appropriate.

The following references relate to the sequences and tissue distribution of PPARs: Auboeuf et al (1997) Diabetes 46(8), 1319-1327; Braissant et al (1996) Endocrinol 137(1), 354-366; Mukherjee et al (1994) J Steroid Biochem Mol Biol 51,157-166; Mukerjee et al (1997) J Biol Chem 272, 8071-8076.

The following references and GenBank Accession numbers relate to the sequences and/or tissue distribution of the indicated polypeptides.
U63846 Human COX-1 cDNA (PTSG1); Hla (1996) Prostaglandins 51, 81-85. NM000963 Human COX2 cDNA (PTSG2); Hla & Neilson (1992) PNAS 89(16), 7384-7388; Jones et al (1993) J Biol Chem 268(12), 9049-9054; Appleby et al (1994) Biochem J 302, 723-727; Kosaka et al (1994) Eur J Biochem 221(3), 889-897.
AF306566 Human phospholipase A2 (secreted form); Valentin et al (2000) Biochem Biophys Res Commun 279(1), 223-228.
NM021628 Human lipogenase ALOXE3.
XM005818 Human lipoxygenase ALOXE5.
XM008328 Human lipoxegenase ALOX12.
NM001141 Human lipoxegenase ALOX15; Brash et al (1997) PNAS 94(12), 6148-6152.
NM005090 and NM 003706 Human phospholipase A2 (cPLA2-gamma) Underwood et al (1998) J Biol Chem 273(34), 21926-21932 and Pickard et al (1999) JBiol Chena 274(13), 8823-8831
M68874 Human phospholipase A2 (cPLA2) Sharp et al (1991) J Biol Chem 266(23), 14850-14853.

It will be appreciated that such a compound may be an agonist or antagonist of the PPAR polypeptide used in the screen and that the intention of the screen is to identify compounds that act as agonists or antagonists of the PPAR, even if the screen makes use of a binding assay rather than an activity assay, for example transcription factor activity or nucleic acid (for example DNA) binding activity. It will be appreciated that the action of a compound found to bind the PPAR polypeptide may be confirmed by performing an assay of transcription factor activity or nucleic acid binding activity in the presence of the compound.

Likewise, such a compound may be an inhibitor or activator of the lipid metabolising or binding entity used in the screen and that the intention of the screen is to identify compounds that act as inhibitors or activators of the lipid metabolising or binding entity, even if the screen makes use of a binding assay rather than an activity assay, for example lipid metabolising activity, for example prostaglandin production from arachidonic acid for COXI or COXII. It will be appreciated that the action of a compound found to bind the lipid metabolising or binding entity may be confirmed by performing an assay of the appropriate enzyme or binding activity in the presence of the compound.

It is preferred that the assay is capable of being performed in a "high throughput" format. This may require substantial automation of the assay and minimisation of the quantity of a particular reagent or reagents required for each individual assay. A scintillation proximity assay (SPA) based system, as known to those skilled in the art, may be beneficial. Combinatorial chemistry techniques may be used in generating compounds to be tested.

A further aspect of the invention provides a kit of parts of screening system comprising (1) a perfluoroctanoic acid or a salt (preferably other than ammonium salt, preferably other than 75% straight chain ammonium salt) or an ester thereof; perflurosuberic acid, perfluoroheptanoic acid, perfluorohexanoic acid, perfluoropentanoic acid, perfluorobutanoic acid or perfluoropropionic acid or a salt or an ester any thereof; or perfluoroctane (2) a PPAR polypeptide or polynucleotide encoding a PPAR polypeptide, and/or a test mammal. The kit may optionally comprise reagents useful in measuring plasma insulin, glucose, triglyceride and/or cholesterol levels, or in measuring PPAR activity, for example nucleic acid binding. Such reagents will be apparent to those skilled in the art, and may include reagents useful in performing transactivation assays or DNA binding assays. Suitable reagents include those described in Example 1.

A further aspect of the invention provides a kit of parts of screening system comprising (1) a perfluoroctanoic acid or a salt (preferably other than ammonium salt, preferably other than 75% straight chain ammonium salt) or an ester thereof; perflurosuberic acid, perfluoroheptanoic acid, perfluorohexanoic acid, perfluoropentanoic acid, perfluorobutanoic acid or perfluoropropionic acid or a salt or an ester any thereof; or perfluoroctane (2) a lipid metabolising or binding entity (for example COXI or COXII or phospholipase A2 or lipoxygenase) or polynucleotide encoding a lipid metabolising or binding entity. The kit may optionally comprise reagents useful in measuring plasma insulin, glucose, triglyceride and/or cholesterol levels, or in measuring the activity of the lipid metabolising or binding entity, for example a substrate of the lipid metabolising or binding entity (for example arachidonic acid in the case of COXII or lipoxygenase) or reagent useful in measuring a product of a lipid metabolising enzyme, for example in assessing eicosanoid biosynthesis. As well known to those skilled in the art, reagents may include labelled ligand, for example radiolabelled or fluorescently labelled. Direct binding or displacement of ligand may be measured. Binding may be measured using fluorescence resonance energy transfer (FRET) techniques. The kit may optionally include reagents useful in cell differentiation assays, for example adipocyte differentiation assays, as will be known to those skilled in the art.

The perfluoroctanoic acid or a salt (preferably other than ammonium salt, preferably other than 75% straight chain ammonium salt) or an ester thereof; perflurosuberic acid, perfluoroheptanoic acid, perfluorohexanoic acid, perfluoropentanoic acid, perfluorobutanoic acid or perfluoropropionic acid or a salt or an ester any thereof; or perfluoroctane useful in the present invention (for example in a method of treatment or in the manufacture of a medicament, as indicated above) may preferably be a compound identified using a screening method as indicated above as having relevant desirable properties.

A perfluoroctanoic acid or a salt (preferably other than ammonium salt, preferably other than 75% straight chain ammonium salt) or an ester thereof; perflurosuberic acid, perfluoroheptanoic acid, perfluorohexanoic acid, perfluoropentanoic acid, perfluorobutanoic acid or perfluoropropionic acid or a salt or an ester any thereof; or perfluoroctane (for example as identified or identifiable by a screening method of the invention is also provided for use in the manufacture of a composition for use as a food supplement or a food additive. The invention also relates to a food product comprising a foodstuff and a perfluoroctanoic acid or a salt (preferably other than ammonium salt) or an ester thereof; perflurosuberic acid, perfluoroheptanoic acid, perfluorohexanoic acid, perfluoropentanoic acid, perfluorobutanoic acid or perfluoropropionic acid or a salt or an ester any thereof; or perfluoroctane (for example identified or identifiable by a screening method of the invention), wherein the food is not laboratory rodent, for example rat or mouse, feed. It is preferred that the food is not laboratory animal feed.

Preferably, the food (the term including food product and foodstuff) is suitable for administration to an animal (for example a domesticated animal as discussed above but not a laboratory rodent) or human, for example an adult human, baby or infant.

The compounds may be administered in any suitable way, usually parenterally, for example intravenously, intraperitoneally or intravesically, in standard sterile, non-pyrogenic formulations of diluents and carriers. The compounds may also be administered topically. The compounds of the invention may also be administered in a localised manner, for example by injection. Preferably, the compounds are administered orally. The compounds may be administered as a tablet or capsule or as a supplement added to food or drink. A slow-release formulation may be used.

All references noted herein are hereby incorporated by example.

The invention is now described in more detail by reference to the following, non-limiting, Figures and Examples:
**Figure 1:** Body weights of animals administered test compounds daily by oral gavage.
**Figure 2:** Terminal body weighs of animals administered test compounds. Values are Mean ±SD. Significantly different from control group: *p<0.05; **p<0.01;***p<0.001.
**Figure 3:** Food consumption (panel A) and food consumed per unit body weight (panel B)of rats treated with test compounds.

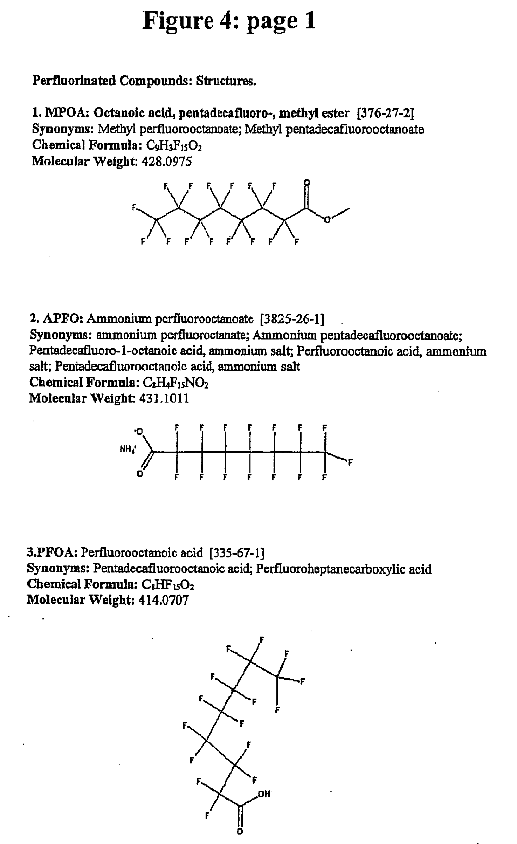

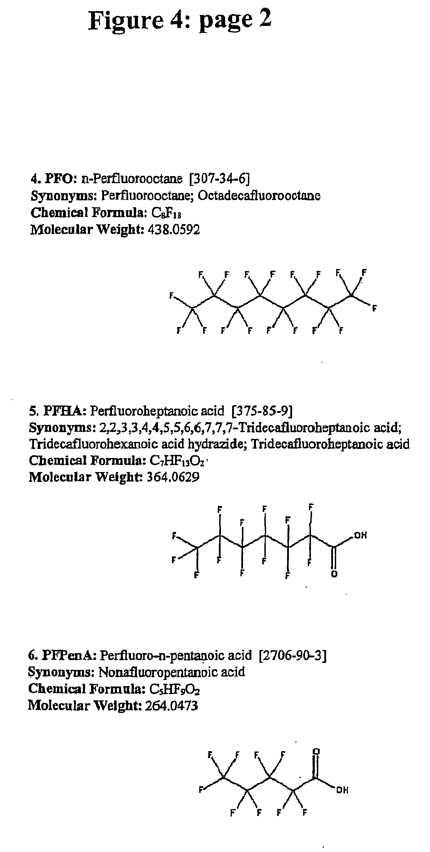

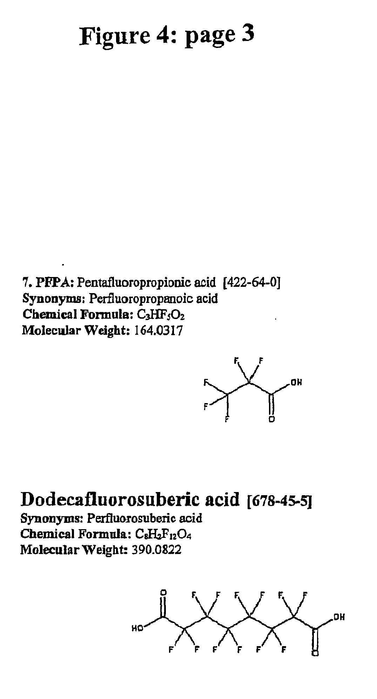

**Figure 4:** Structures of test compounds.

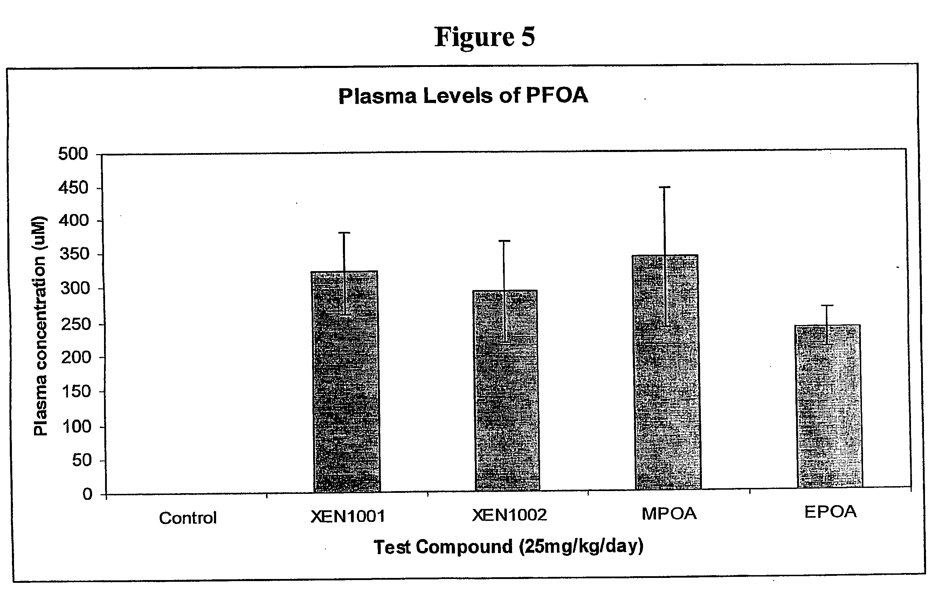

**Figure 5:** Plasma concentrations of APFO following administration of Test compound at 25mg/kg daily for 11 days. Values are expressed as Mean ± SD, n=8 (control) or 4 (test). Significantly different from control: p≤0.001**, p≤0.001***.

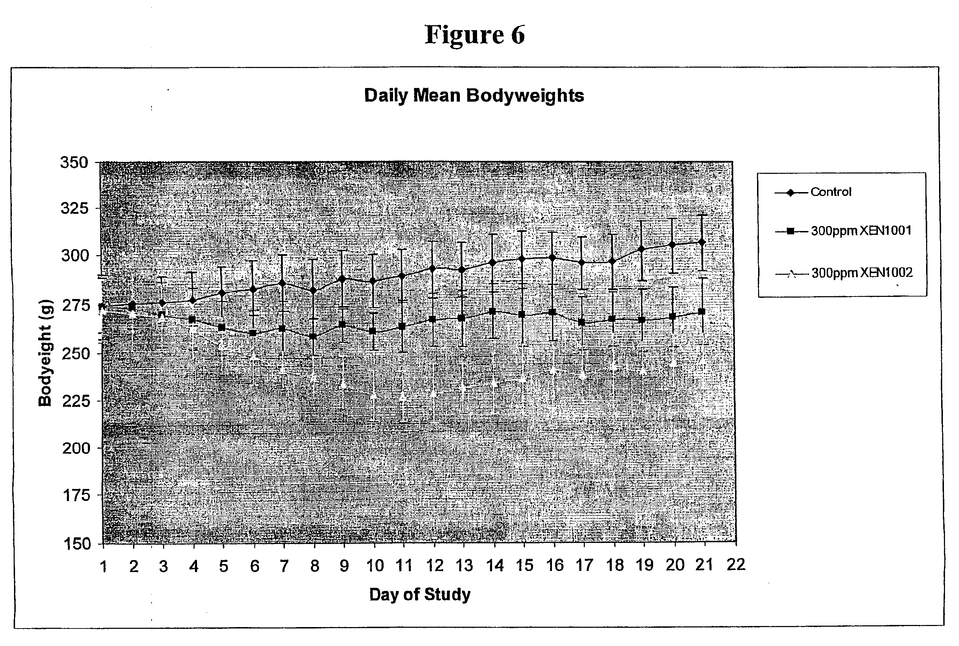

**Figure 6:** Daily bodyweights of rats exposed to test compounds administered in the diet daily for 21 days. Values are expressed as Mean ± SD, n=4.

### Example 1: Biological effects of perfluorinated compounds

A variety of studies were performed
1. To establish the therapeutic potential of particular perfluorinated compounds in the treatment of diabetes and obesity.
2. To establish the potential anti-cancer activity of particular perfluorinated compounds in cultured tumour cells
3. To establish the interactions of particular perfluorinated compounds with peroxisome proliferator activated receptors.

### Test Compounds

The biological effects of particular perfluorinated compounds were assessed using a variety of *in vitro- and in vivo*- based investigations. Compounds studied included a salt (ammonium perfluorooctanoate), acids containing 8 carbons (perfluorooctanoic acid and perfluorosuberic acid [dicarboxylic acid]), acids with chain lengths of 7, 5 and 3 carbon atoms (perfluoroheptanoic acid, perfluoropentanoic acid and perfluoropropionic acid respectively), an ester (methylperfluorooctanoate) and an alkane (perfluorooctane). Ammonium perfluorooctanoate was obtained from 3M; all other perfluorinated compounds were purchased from Sigma.

**Table 1: Perfluorinated compounds and their corresponding CAS numbers**

| ***Compound*** | **CAS No.** |
|---|---|
| Ammonium perfluorooctanoate (APFO) | 3825-26-1 |
| Perfluorooctanoic acid (PFOA) | 335-67-1 |
| Methyl perfluorooctanoate (MPOA) | 376-27-2 |
| Perfluorooctane (PFO) | 307-34-6 |
| Perfluorosuberic acid (C8) (PFSA) | 678-45-5 |
| Perfluoroheptanoic acid (PFHA) | 375-85-9 |
| Perfluoropentanoic acid (PFPenA) | 2706-90-3 |
| Perfluropropionic acid (PFPA) | 422-64-0 |

### 2.In vitro Studies

### 2.1 Growth Inhibition Studies

The effect of perfluorinated compounds on the inhibition of tumour cell growth was assessed *in vitro* using three human tumour-derived cell lines. Cancer cell lines were tested using the sulphorhodamine B (SRB) assay (which measures cellular protein and is directly related to cell number), which is utilised by the National Institute of Cancer/National Institute of Health (NIC/NIH) to screen anti-cancer agents.

### 2.1.1 Experimental Design and Methods

HT-29 cells (human colon tumour-derived), MCF7 cells (human breast cancer-derived) and PC3 cells (human prostate cancer-derived) were harvested and diluted (to 5 x 10⁴ cells per ml, 1x 10⁵ cells per ml and 7.5 x 10⁴ cells per ml respectively) in RPMI 1640 medium containing 5% FBS, 2mM glutamine and 50µg/ml gentamicin. 100µl of cell suspension per well was added to 96 well plates and allowed to attach overnight. Cells were exposed to test compounds dissolved in DMSO in 100µl of medium to give final drug concentrations of 0, 0.3, 1, 3, 10, 30, 100,300, 1000 and 3000µM (and DMSO at 0.25%). (n = 6 for each drug concentration). After 48 hours, cells were fixed in 10% trichloroacetic acid (TCA) at 4°C for 1-2 hours then washed using water and subsequently air-dried. 100µl SRB solution (0.4%) in 1% Acetic Acid was added to each well, and plates were incubated at room temperature for 10 minutes. Unbound dye was removed by washing with 1 % acetic acid. Plates were air-dried and bound stain was solubilised in 10mM Tris base and the absorbance read at 520nm. The concentration at which 50% inhibition of cell growth (IC₅₀) was achieved was calculated using Graphpad Prism software and tabulated.

### 2.1.2 Results and Discussion.

A number of compounds proved to have little effect on growth inhibition, with IC₅₀ values that were close to, or exceeded, 3000µM. These were the ester derivative (MPOA), the dicarboxylic form (PFSA) and acids with chain lengths of 5 or less (PFPenA and PFPA). The IC₅₀ values of all other compound (APFO, PFOA and PFHA) were within the 200-600µM range.

**Table 2: IC₅₀ values of perfluorinated compounds on three human tumour cell lines.**

| 1.1.1 Compound | **IC₅₀** | | |
|---|---|---|---|
| | **HT-29** | **MCF7** | **PC3** |
| **APFO** | 293 µM | 211 µM | 215 µM |
| **PFOA** | 398 µM | 244µM | 215µM |
| **MPOA** | >3000µM | >2786µM | >3000µM |
| **PFSA** | >3000µM | >3000µM | >3000µM |
| **PFHA** | 371µM | 573µM | 321µM |
| **PFPenA** | >3000µM | >3000µM | 2630µM |
| **PFPA** | >3000µM | >3000µM | >3000µM |

### 2.1.3 Conclusions

This study indicated that the ammonium salt and the acid form of the compound were effective at inhibiting the growth of a range of human tumours within similar concentration ranges. The number of carbon atoms within the compound also had an effect on cells growth, with 5 carbons and below having little consequence on cellular protein levels. The dicarboxylic acid and methyl ester were also non-toxic to those cancer cell lines exposed to the compounds.

### 2.2 Interaction of Perfluorinated Compounds with PPAR Isoforms

Transactivation assays involving human PPAR gamma and delta and rat alpha cDNA, and ligand binding assays using human PPAR gamma, were performed in order investigate the interaction of the compounds with PPAR isoforms.

### 2.2.1 PPAR Transactivation Assays: Experimental Design and Method

COS-1 cells (African green monkey kidney cells), [cultured in Dulbecco's Modified Eagles Medium (DMEM) supplemented with 10% heat-inactivated foetal calf serum, 2mM L-glutamine, penicillin (50IU/ml), and streptomycin (50 µg/ml)], were plated into 12 well tissue culture dishes at 1.5 x 10⁵ cells per well and allowed to adhere overnight at 37°C. The next day the medium was aspirated and the cells washed with PBS, pH7.4, and 100µl of a transient transfection cocktail was added to each well. The transfection cocktail was composed of 25ng of vector DNA carrying PPAR alpha, delta or gamma, 250ng of plasmid DNA containing the PPAR response element of liver fatty acid binding protein and firefly luciferase, and, as a transfection control, 250ng of a vector harbouring (β-Galactosidase. As a negative control, cells were transfected with the reporter vectors only; cells were also exposed to a transfection cocktail that contained no plasmid DNA. DNA was dissolved in PBS containing 50µg.ml DEAE-Dextran. Cells were incubated at 37°C for 30 minutes before 1ml of medium containing 80µM chloroquine was added and the cells incubated for a further 2.5 hours at 37°C. The medium was aspirated and the cells shocked with 0.5ml 10% DMSO in medium for 2.5 minutes at room temperature. Cells were washed with PBS then allowed to recover at 37°C in growth medium for 48 hours.

Transiently transfected cells were exposed to perfluorinated compounds (dissolved in DMSO) in medium at 0, 0.01, 0.1, 0.3, 1, 3, 10, 30, 100, 300 and 1000µM for 24 hours at 37°C. Additionally, transfected cells were exposed to Wyeth14643, carbaprostacyclin or rosiglitazone, which were used as positive controls for PPAR- alpha, -delta and -gamma respectively. Cells were then washed, lysed, and luciferase and (β-Galactosidase activities were measured using kits according to the methods specified by the manufacturer (Promega, Madison, USA) by flash luminescence and spectophotometry respectively. Luciferase expression was normalised by dividing by the flash luminescence reading with constitutive β-Galactosidase expression levels measured at 415nm following a colourimetric assay. To correct for background expression levels, values obtained from cells transfected with reporter plasmids alone were subtracted from test readings.

Data were graphed, fitted to non-linear regression curves and fold activation values calculated using GraphPad Prism software.

### 2.2.1.1. Results and discussion

PPAR alpha was activated 11.25-fold by APFO compared to values obtained from cells exposed to vehicle only. PFO, PFHA and PFPenA activated PPAR alpha approximately 5-fold; MPOA and PFPA activated the receptor approximately 2-fold. While there was no activation of PPAR alpha by PFSA, this compound did activate PPAR gamma approximately 4-fold. Activation of PPAR gamma occurred within the range ~2- 6-fold, with APFO and PFPenA at the top of the range. There was no activation of PPAR delta by any of the perfluorinated compounds. However, there was inhibition of activation of PPAR delta by PFOA (by a factor of 4), PFHA and PFPenA (by a factor of 2.2), and, to a lesser extent, PFPA and PFSA (by a factor of 1.6) (table 3).

**Table 3: Fold activation of PPAR isoforms.**

| | *Positive Control* | **MPOA** | **APFO** | **PFOA** | **PFHA** | **PFPenA** | **PFPA** | **PFSA** |
|---|---|---|---|---|---|---|---|---|
| **Alpha** | 8.88±1.94 (10µM) | 2.50±0.28 | 11.25±300 | 5.14±1.89 | 4.55±1.31 | 4.19±0.15 | 136±0.51 | 0.65±0.07 |
| **Gamma** | 3.39±1.12 | 2.92±0.12 | 4.11±1.51 (1.25± 0.38) (3 µM) | 3.59±2.74 | 3.43±2.62 (1.45± 0.19) (10 µM) | 5.07±2.29 (1.45± 0.19) (10 µM) | 1.96±0.81 | 3.75±1.03 |
| **Delta** | 2.17±0.42 | 1.51±0.31 | 1.04±1.18 | 0.25±0.05 | 0.45±0.28 | 0.45±0.04 | 0.63±0.25 | 0.62±0.31 |

Fold activation was measured at 1mM unless otherwise indicated in the table. If maximal activation by the test compound occurred below 1mM, the corresponding fold activation by the positive control, and the relevant concentration, are shown in parenthesis. Values are expressed as the mean ± SD (n=3).

### 2.2.1.2 Conclusions

These results indicate that the compounds activate both PPAR alpha and PPAR gamma receptors, while some (PFSA) appear to interact specifically with a single isoform of the receptor. Inhibition of activation of PPAR delta suggests that perfluorinated compounds may be PPAR delta antagonists, which may be important with respect the treatment of cancer (as PPAR delta expression levels have been shown to be increased in colorectal cancer cells [He, TC, Vogelstein, B and Kinzler, KW. Cell 99: 335-345 (1991)]) and atherosclerosis (as PPAR delta induces lipid accumulation in macrophages [Vosper, H et al. J. Biol. Chem. 276: 44258-44265 (2001].

### 2.2.2 PPAR Ligand Binding Studies: Experimental Design and Methods

GST-tagged human PPAR-alpha and -gamma ligand binding domains were expressed in *E. Coli.* Briefly, the ligand binding domains, comprising amino acids 179-468 of PPAR alpha and amino acids 174-478 of PPAR gamma, were generated by PCR (using oligonucleotides that contained the required restriction enzyme sites) and cloned into the vector pCR-Bluntll-TOPO (Invitrogen Ltd., Paisley, UK). Domains were excised using Nco/Xhol and cloned into the vector pGEX6PB (Amersham Biosciences, Bucks., UK), which contained a bacterial GST-tag, and transformed into *E.coli* by standard methods. Verified clones were cultured overnight at 37°C under ampicillin selection. Overnight cultures were used to inoculate fresh culture medium at 1:100 and protein expression was induced with 0.5mM β-D-isopropylthiogalactopyranoside (IPGT) for 3 hours at 30°C when cells were in mid-log phase. Soluble protein from the induced cultures was harvested and purified by affinity chromatography against glutathione agarose (Sigma, Poole, UK) and eluted using 50mM Tris-HCl, pH8.0/5mM Glutathione (reduced form).

Recombinant receptor proteins have been used previously to study interactions with the fluorescent fatty acid - *cis*-parinaric acid (CPA)[Palmer CAN and Wolf CR. FEBS Letts. 431, 476-480, (1998); Causevic M, Wolf CR and Palmer CAN. FEBS Letts. 463, 205-210, (1999)]). Upon binding to the receptor, changes in the spectral properties of the fatty acid occur. These are quantitatively related to the binding of the ligand to the receptor and can be used to calculate binding constants. Perfluorinated compounds were assayed for their ability to displace 11-(5-dimethylaminonapthalenesulphonyl)-undecanoic acid (DAUDA) from PPAR alpha and cis-parinaric acid from PPAR gamma. Data were analysed as described in section 2.2.1. (Table 4)

### 2.2.2.1 Results and Discussion.

The ammonium salt (APFO) bound PPAR alpha with a similar affinity to PFOA, with apparent K_{d} values of 8µM and 6µM respectively. The dicarboxylic acid (PFSA) and the 5 carbon compound (PFPenA) also had similar binding affinities of 44µm and 62µm respectively, which translates to 7- and 10- fold reductions in binding affinity for the isoform compared to PFOA. The 7 carbon compound had the lowest binding affinity for PPAR alpha, with a K_{d} of 215µm (a 30-fold reduction in ligand binding compared to PFOA), while PFPA (3 carbons) had increased affinity compared to PFOA, with a K_{d} of 2µM (Table 4).

Addition of the ammonium group reduced the binding affinity of the compound to PPAR gamma by a factor of ten, with K_{d} values of 0.3µM and 3µM for PFOA and APFO respectively. Reducing the number of carbon atoms from 8 to 5 (pFPenA) and 3 (PFPA) reduced PPAR gamma ligand binding affinity approximately 50- (17µM) and 2-fold (0.6µM) respectively, while the 7 carbon compound (PFHA) showed reduced ligand binding affinity, with a K_{d} of 99µM. The dicarboxylic acid (PFSA) demonstrated reduced affinity for PPAR gamma ligand binding by a factor of ~40 (13µm) (Table 4).

**Table 4: Ligand Binding of Perfluorinated Compounds (Apparent K_{d}))**

| | PPAR Receptor Binding K_{d} (µM) | |
|---|---|---|
| Compound | Alpha | Gamma |
| *APFO* | 8 | 3 |
| PFOA | 6 | 0.3 |
| PFSA | 44 | 13 |
| PFHA | 215 | 99 |
| PFPenA | 62 | 17 |
| PFPA | 2 | 0.6 |

### 2.2.2.2 Conclusions

These results suggest that perfluorinated compounds bind to human PPAR alpha and gamma ligand binding domains *in vitro.*

### 3. In Vivo Studies.

### 3.1 Effects of perfluorinated compounds on metabolic parameters in male CR Sprague Dawley (CR SD) rats.

In previous studies, APFO was shown to possess anti-diabetic and anti-obesity capabilities in both healthy and disease models of these conditions. In order to demonstrate that other perfluorinated compounds possessed the same therapeutic potential, selected perfluorinated compounds were administered to SD rats for 7 days and physical and biochemical parameters monitored.

### 3.1.1 Experimental Design and Methods.

Two groups (n=5) of CR SD rats (approximately 300g) were treated with 2 dose levels of perfluorinated compounds (15 and 25mg/kg/day body weight). Animals were administered test substances, dissolved in corn oil, by oral gavage, daily for 7 days. One group of 20 CR SD rays was also treated with vehicle (corn oil) alone. Body weight and food consumption were monitored daily.

Twenty-four hours after the last dose the animals were killed by an increasing concentration of carbon dioxide. Blood was collected by cardiac puncture and plasma was prepared and stored at -70°C until analysed.

Plasma was analysed for triglycerides, cholesterol, and glucose, using kits purchased from Sigma. Concentrations of plasma insulin were determined using a commercially available enzyme-immunoassay-based kit from Amersham Biosciences. All assays were carried out as specified by the manufacturer.

### Results and Discussion

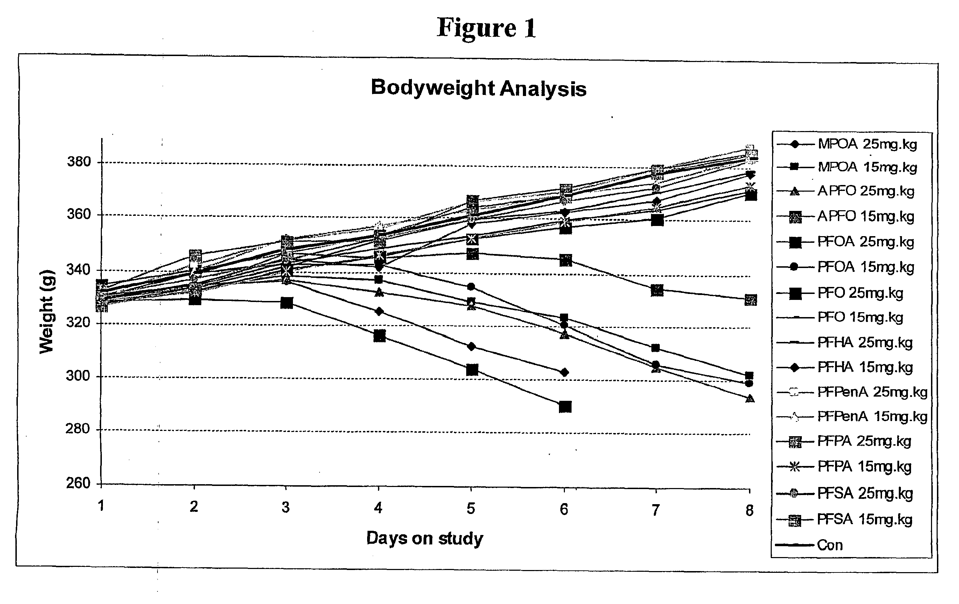

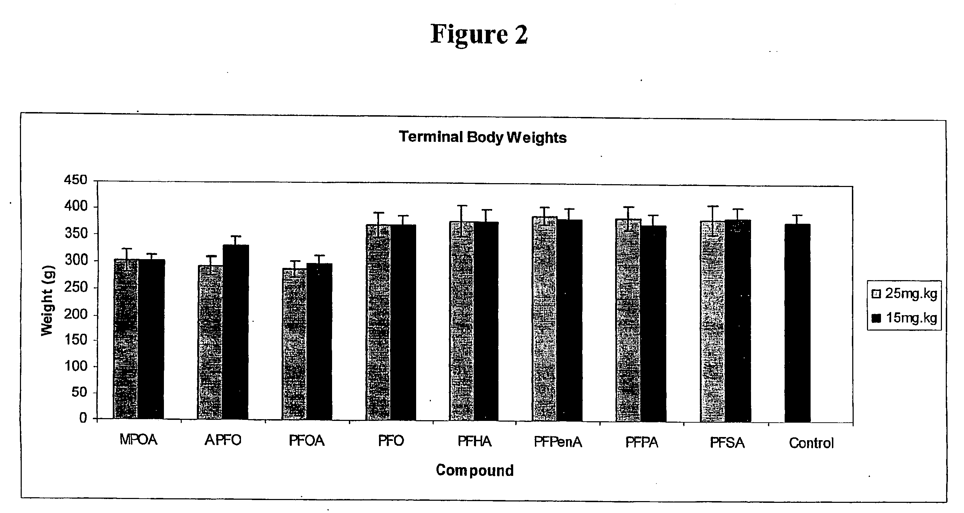

Rats treated with APFO, MPOA and PFPA at 15-and 25mg/kg/day lost bodyweight after day 3, and this continued till the end of the treatment period (Figure 1). Animals administered 25mg/kg MPOA and PFOA were terminated early, having lost 8% and 11.7% of their bodyweight respectively after day 6. Weight loss in animals dosed with 15mg/kg of these 3 compounds was less pronounced: 1% (compared with 11% at the higher dose) with APFO, 8.2% and 9.4% for MPOA and PFOA respectively. No weight loss was observed in animals treated with the remaining compounds, with animals gaining 12-18% bodyweight over the test period (Figure 2).

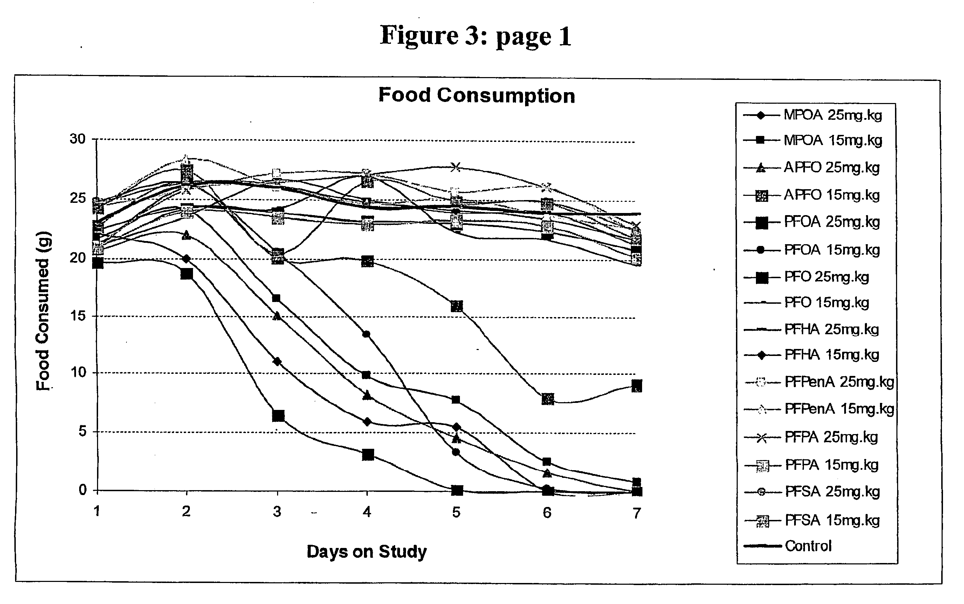

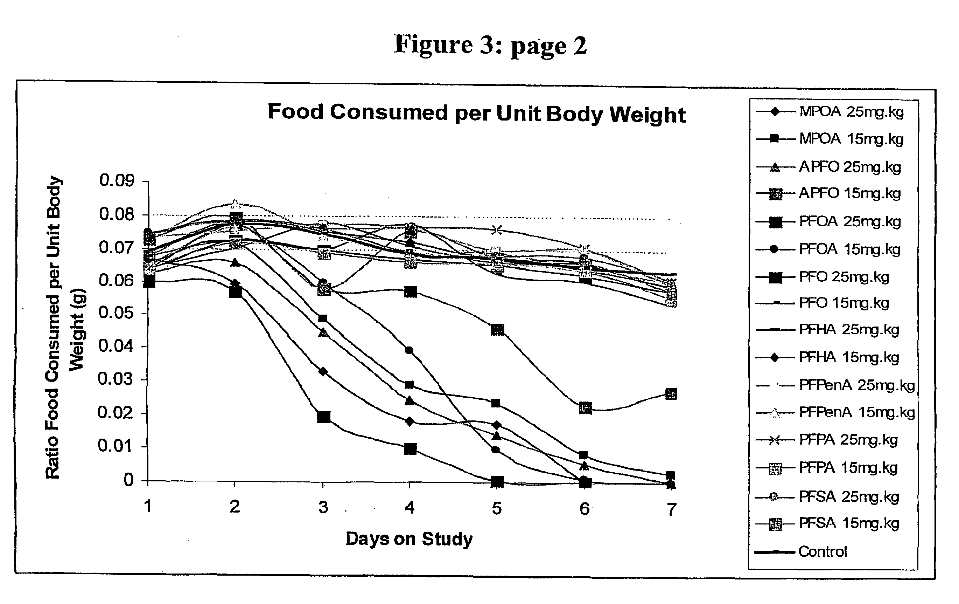

Body weight losses in rats treated with MPOA, APFO and PFOA were reflected in dose-related, decreases in food consumption (Figure 3A). When expressed in terms of food consumed per gram bodyweight the pattern of effect was similar (Figure 3B). Food consumption in rats administered 15mg/kg APFO did not decrease further after day 6, and this was also observed when data were expressed as food consumed per unit body weight. In all other test groups, food consumption was similar to that seen in control animals.

Insulin levels were reduced in the plasma of all rats treated with perfluorinated compounds, but this was not dose-dependent, suggesting that a maximum pharmacological effect was achieved at the lowest dose studied. The most marked reductions were seen in the plasma of animals administered MPOA, APFO and PFOA. At 25mg/kg, plasma insulin concentrations were reduced tol5.6% (2.6% at 15mg/kg), 8.3% (13.5% at 15mg/kg) and 30.74% (2.98% at 15mg/kg) of control values. Significant reductions were also observed in the plasma of rats administered 25mg/kg PFO and PFPenA, and in animals dosed with PFSA at 15mg.kg, with levels reduced to 33.5%, 33.9% and 28.7% of control values respectively. At 15mg/kg, plasma levels in rats dosed with PFHA and PFPA were reduced by approximately 30-40%, but this was not statistically significant (Table 5).

Plasma glucose was reduced in animals administered MPOA, APFO and PFOA to 78.6%, 72.6% and 60% of control values respectively, and, again, these reductions were not dose-dependent. Levels were raised by 3% in the plasma of animals dosed with 25mg/kg PFO. No other significant changes were observed in glucose levels (Table 5).

The most significant (non-dose-related) reductions in plasma triglycerides were again seen in those animals administered MPOA, APFO and PFOA to 33.7%, 19.1% and 16.8% of control values respectively at 25mg/kg. All other compounds reduced triglycerides, but differences here were less pronounced (42.1% - 68.5% of control values) (Table 5).

Plasma cholesterol was significantly reduced in a non-dose-related fashion by all compounds with the exception of PFSA and PFO. At 25mg/kg, values dropped to 36.7%, 56.2% and 49.2% of control values for MPOA, APFO and PFOA respectively. Reductions of cholesterol in animals treated with the remaining compounds dropped to 69.8%-85% of control levels (Table 5)

**Table 5: Metabolic parameters in SD rats administered with perfluorinated compounds. Values are Mean ± SD. (n=5 for test groups; n=20 for control group) Significant difference from control group is indicated.**

| *Compound* | **Insulin (ng/ml)** | | **Glucose (mmol/l)** | | **Triglycerides (mmol/l)** | | **Cholesterol (mmol/l)** | |
|---|---|---|---|---|---|---|---|---|
| | **25mg.kg** | **15mg.kg** | **25.m.gk** | **15mg.kg** | **25mg.kg** | **15mg.kg** | **25mg.kg** | **15mg.kg** |
| *MPOA* | 28.74 | 4.87 | 12.99 | 11.48 | 0.6 | 0.28 | 0.73 | 1.17 |
| | ±17.22 | ±2.611 | ±2.07 | ±1.27 | ±0.89 | ±0.08 | ±0.23 | ±0.31 |
| | p<0.001 | p<0.001 | p<0.05 | p<0.001 | p<0.05 | p<0.001 | p<0.001 | p<0.01 |
| **APFO** | 15.29 | 25.02 | 11.99 | 13.12 | 0.34 | 0.25 | 1.12 | 1.00 |
| | ±9.72 | ±20.27 | ±1.87 | ±0.74 | ±0.09 | ±0.07 | ±0.37 | ±0.36 |
| | p<0.001 | p<0.001 | p<0.01 | p<0.001 | p<0.001 | p<0.001 | p<0.01 | p<0.01 |
| **PFOA** | 56.76 | 5.51 | 10.01 | 10.30 | 0.3 | 0.37 | 0.98 | 1.01 |
| | ±46.5 | ±8.88 | ±3.90 | ±1.23 | ±0.13 | ±0.04 | ±0.35 | ±0.086 |
| | p<0.01 | p<0.001 | p<0.05 | p<0.001 | p<0.001 | p<0.001 | p<0.001 | p<0.001 |
| **PFO** | 61.81 | 99.58 | 17.13 | 18.41 | 2.09 | 1.03 | 1.70 | 1.99 |
| | ±45.81 | ±126.1 | ±2.37 | ±1.41 | ±2.33 | ±0.35 | ±0.11 | ±0.46 |
| | p<0.01 | | | p<0.05 | | p<0.05 | P<0.01 | |
| **PFHA** | 165.1 | 114.2 | 18.15 | 16.43 | 0.75 | 0.94 | 1.46 | 1.50 |
| | ±114.4 | ±90.06 | ±2.78 | ±2.59 | ±0.26 | ±0.20 | ±0.17 | ±0.23 |
| | | | | | p<0.001 | p<0.01 | p<0.001 | p<0.01 |
| **PFPenA** | 62.6 | 124.7 | 16.67 | 16.22 | 1.07 | 1.24 | 1.67 | 1.83 |
| | ±68.26 | ±126.3 | ±0.93 | ±1.29 | +0.37 | ±0.04 | ±0.20 | ±0.41 |
| | p<0.05 | | | | p<0.05 | p<0.05 | p<0.05 | |
| **PFPA** | 179.7 | 129 | 14.97 | 15.46 | 0.95 | 1.02 | 1.39 | 1.28 |
| | ±177.1 | ±36.05 | ±1.29 | ±1.13 | ±0.17 | ±0.38 | ±0.39 | ±0.10 |
| | | | | | p<0.01 | p<0.05 | p<0.05 | P<0.001 |
| **PFSA** | 109 | 52.96 | 15.9 | 16.41 | 1.22 | 0.95 | 1.92 | 1.76 |
| | ±103.2 | ±26.35 | ±1.43 | ±2.09 | ±0.56 | ±0.42 | ±0.36 | ±0.15 |
| | | p<0.01 | | | | p<0.05 | | |
| **Control** | 184.6 | | 16.52 | | 1.78 | | 1.99 | |
| | ±124.7 | | ±2.60 | | ±1.06 | | ±0.36 | |

### 3.1.2 Conclusions.

In summary, there were a number of significant physiological effects that could be related to the administration of perfluorinated compounds. The results of these studies indicated that changing the structure of perfluorinated compounds had a number of beneficial effects.

Only three compounds (MPOA, APFO and PFOA) caused weight loss and reduced food consumption, while the animals appeared otherwise normal (which may be of use in the treatment of obesity). Clinical parameters were altered in animals administered with perfluorinated compounds that did not result in weight loss, suggesting that biochemically important factors (such as insulin levels) could be targeted without affecting bodyweight. All compounds tested reduced triglycerides and all, with the exception of PFSA and PFO, reduced cholesterol, suggesting that they may be of therapeutic use in the treatment of hyper-lipidaeamia and -cholesterolaemia. Reductions in the levels of insulin (by all compounds, suggesting clinical use as insulin sensitizers) and glucose by APFO, PFOA and MPOA indicate that these chemicals may be of therapeutic use for the treatment of Type II diabetes.

APFO, MPOA and PFOA all had the same insulin-, glucose-, cholesterol- and triglyceride-lowering and weight loss effects *in vivo. In vitro,* however, MPOA had little impact on the inhibition of cell growth and had comparatively lower affinity for PPAR isoforms according to data from transactivation assays. This 'dampened' response may have been due to the methyl group. *In vivo,* this moiety may have been removed by esterases (which are present in high concentrations in plasma), leaving a compound that is similar to PFOA. APFO, too, is metabolised *in vivo* by the removal of the ammonium group, again producing a compound essentially the same as PFOA. The presence of the ammonium salt on APFO increased affinity of the compound to PPAR alpha in a transactivation assay; inhibition of cell growth occurred within the same concentration range as that caused by PFOA, suggesting that a structural feature common to the two molecules was responsible for the inhibition of growth of tumour cells *in vitro.*

In transactivation assays PFSA no longer bound PPAR alpha, but did retain PPAR gamma activity. This suggested that the addition of a CO₂ group to PFOA altered PPAR specificity; this also essentially reduced the effect on insulin and triglycerides, and removed the effect on glucose and cholesterol.

In transactivation assays PFHA (a C7 acid) retained PPAR specificity for PPAR alpha, delta and gamma but showed a slightly reduced anti-tumour potency. Additionally, insulin sensitisation by the compound was reduced, but the effects on triglycerides and, to a lesser extent, cholesterol, were retained. Reduction of the chain length to C7 retained the effects on triglycerides without causing anorexia, and this did not appear to be via effects on insulin.

PFPenA (a C5 acid) retained PPAR specificity in the cell-based assay and lowered insulin without affecting triglyceride levels. The compound also did not inhibit the growth of tumour cells *in vitro.* Reduction of the chain to C3 (PFPA) essentially removed all *in vitro* effects, and removed insulin and glucose-lowering capabilities while reducing the effect on triglycerides and cholesterol.

PFO (absence of CO₂H) could not be tested *in vitro,* as the compound was insoluble. *In vivo,* however, PFO still demonstrated insulin sensitising capabilities but had no effect on lipids.

In summary, modifications to PFOA had a number of effects *in vitro* and *in vivo.* Carbon chain length was shown to alter the compounds' properties. Generally, the greater the number of carbons in the chain, the greater was the anti-tumour effect (C8>C7>C5=C3=0). Chain length also affected PPAR alpha affinity (C8-NH₄>C8=C7=C5>C3=0), PPAR gamma agonism (C8-NH4=C5>C8>C7>C3), and PPAR delta antagonism (C8>C7=C5>C3) according to transactivation studies. Finally, with the exception of the dicarboxylic acid (PFSA), compounds containing less than 8 carbon atoms did not cause weight loss or reduced food consumption.

In addition to transactivation assays, ligand-binding studies were also performed in order to show that perfluorinated compounds interacted with PPAR isoforms (alpha and gamma). While the cell-based assay showed activation or inhibition of activation by a compound (indicating agonism and antagonism respectively) of the nuclear receptor, the ligand binding assay did not differentiate between agonism or antagonism of a given compound. Additionally, fold activation observed in a transactivation assay could not be compared with an EC₅₀ value obtained from a ligand binding study. Differences between the two assays need to be accounted for. For example in the transactivation assay cell-specific factors may be at play (e.g. levels of activation may be affected by uptake of the compound). Comparison between the two assays can be, therefore, difficult.

### Example 2: Effects of perfluorinated esters and compounds differing in extent of linearity

The objectives of these experiments were:
To establish the therapeutic potential of perfluorinated compounds of different % linearity.
To establish the suitability of perfluorinated esters as pro-drugs.

### Test Compounds

Four perfluorinated compounds were assessed using two *in vivo-* based investigations. Compounds studied were the ammonium salt ammonium perfluorooctanoate in the 100% and 75% linear forms, and two esters (methylperfluorooctanoate and ethylperfluorooctanoate).

**Table 1: Perfluorinated compounds and their corresponding CAS numbers**

| **Compound** | **CAS Number** | **Supplier** | **Description** |
|---|---|---|---|
| Ammoniumperfluorooctano ate 75% linear (XEN1001) | 376-26-1 | 3M Toxicology Services, USA | White Powder with a slight odour |
| Ammoniumperfluorooctano ate 100% linear (XEN1002) | | Miteni, Italy | |
| Methylperfluorooctanoate (MPOA) | 376-27-2 | Sigma Aldrich, UK | Colourless liquid |
| Ethylperfluorooctanoate (EPOA) | 3108-24-5 | | |

### In vivo Studies

### Oral Exposure Study to Measure Plasma Levels of PFOA (perfluorooctanoic acid): Experimental Design and Methods

The study consisted of one control group of 8 male CD Sprague Dawley rats (8-10-weeks old; obtained from Harlan, UK) and 4 test groups that contained 4 rats. Animals received powdered RM1 diet *ad libitum* for the duration of the study. Rats were orally administered XEN1001 (75% linear APFO), XEN1002 (100% linear APFO), MPFO and EPFO at 25mg/kg, daily for 11 days. All chemicals were dissolved in Mazola corn oil (at 2.5mg/ml) and test compounds were administered at 10ml/kg bodyweight. Control animal received Mazola corn oil only. All animals were sacrificed after 11 days. Animals were killed by exposure to rising concentrations of CO₂, and blood was harvested into lithium/heparin tubes for plasma.

Rat plasma samples were denatured by adding acetonitrile, and the amount of perfluorooctanoic acid (PFOA) present was determined by LC/MS/MS.

### Results and Discussion: Plasma Concentration of PFOA Following Administration of Pefluorinated Compounds

Circulating levels of the free acid PFOA were measured 24 hours following the final administration of test compounds. MS analysis showed that rat plasma contained equimolar concentrations of PFOA in rats administered the ammonium salts (XEN1001 or XEN1002) or the methyl (MPOA) or ethyl ester (EPOA) (Figure 1). This suggested that the methyl and ethyl esters were completely metabolised to the free acid (active compound) when administered by oral gavage to SD rats.

### Effect of Perfluorinated Compounds of Different % Linearity on Bodyweights of SD Rats: Experimental Design and Methods

In a previous 7-day study, rats administered XEN1001 showed continued weight loss over the study period. This experiment investigated the effect of compounds of different % linearity on body weight over a longer study period.

The ammonium salts XEN1001 (75% linear APFO) and XEN1002 (100% linear APFO) were added to RM1 powdered diet at 300ppm, equivalent to 0.3g of XEN1001 or XEN1002 per kg of diet. The diet study consisted of one control group of 4 male animals and 4 test groups of 4 male animals (as specified earlier in this Example). Animals received powdered RM1 diet alone, or RM1 diet containing 300ppm XEN1001 or XEN1002, *ad libitum* for the duration of the study. Bodyweights were measured daily for 21 days.

### Bodyweight of SD Rats: Results and Discussion

Rats administered XEN1001 or XEN1002 lost bodyweight up to day 7 and 10 respectively, compared to control animals which steadily gained weight throughout the study. Following this initial loss, test groups gained weight in parallel to control groups without equalling control bodyweights. Weight loss in animals administered XEN1002 in the diet was more pronounced than that seen in animals exposed to XEN1001, suggesting that the 100% linear form of the compound was more active *in vivo* (Figure 2).

This pattern of weight loss followed by recovery of weight gain - which follows the pattern of weight loss accompanied by reduced insulin, glucose, triglycerides and cholosterol seen previously with 75% linear APFO - shows that the 100% linear ammonium salt XEN1002 is more effective at producing a biological response in SD rats.

### Conclusions

Plasma concentrations of PFOA following gavage administration of the esters mirrored those seen in rats dosed with the ammonium salts, suggesting that the methyl and ethyl ester forms of the compound could be utilised as pro-drugs.

Bodyweight analysis demonstrated that administration of the ammonium salts, particularly the 100% linear form, resulted in a biological response.

As all compounds were metabolised to the active free acid in equimolar amounts, administration of PFOA or esters can produce the same biological effects as those produced by the ammonium salts.

Embodiment of the invention include the following:
1. A method of treatment of a patient in need of modulation of body mass or modulation of increase in body mass, and/or in need of modulation of plasma insulin, plasma glucose, plasma triglycerides and/or plasma cholesterol, comprising administering to the patient an effective amount of a perfluoroctanoic acid or a salt (preferably other than ammonium salt, preferably other than 75% straight chain ammonium salt) or an ester thereof; perfluorosuberic acid, perfluoroheptanoic acid, perfluorohexanoic acid, perfluoropentanoic acid, perfluorobutanoic acid or perfluoropropionic acid or a salt or an ester any thereof; or perfluoroctane.
2. A method of treatment of a patient in need of an antitumour agent or an antiinflammatory agent, or in need of modulation in lipid or eicosanoid status, comprising administering to the patient an effective amount of a compound as defined in embodiment 1.
3. A method of treatment of a patient who is overweight or obese and/or has diabetes, hyperlipidaemia, atherosclerosis, coronary heart disease, stroke, obstructive sleep apnoea, arthritis and/or reduced fertility, or is at risk of developing such a condition, comprising administering to the patient an effective amount of a compound as defined in embodiment 1.
4. A method of treatment of a patient in need of modulation of PPAR (for example PPARα) activity, comprising administering to the patient an effective amount of a compound as defined in embodiment 1.
5. A method of treatment of a patient in need of modulation of lipid or eicosanoid status or function, comprising administering to the patient an effective amount of a compound as defined in embodiment 1.
6. Use of a compound as defined in embodiment 1 in the manufacture of a medicament for treating a patient in need of modulation of PPAR (for example PPARα) activity.
7. The method of embodiment 4 or use of embodiment 6 wherein the patient is in need of an increase in PPAR activity and the compound is a PPAR agonist.
8. The method or use of embodiment 7 wherein the PPAR is PPARα or PPARγ.
9. The use of a compound as defined in embodiment 1 in the manufacture of a medicament for the treatment of a patient in need of modulation of body mass or modulation of increase in body mass, and/or in need of modulation of plasma insulin, plasma glucose, plasma triglycerides and/or plasma cholesterol.
10. The method of embodiment 1 or use of embodiment 9 wherein the patient is in need of reduction of body mass or prevention of increase in body mass, and/or in need of reduction of plasma insulin, plasma glucose, plasma triglycerides and/or plasma cholesterol.
11. The use of a compound as defined in embodinent 1 in the manufacture of a medicament for the treatment of a patient who is overweight or obese and/or has diabetes, hyperlipidaemia, atherosclerosis, coronary heart disease, stroke, obstructive sleep apnoea, arthritis and/or reduced fertility, or is at risk of developing such a condition.
12. The use of a compound as defined in embodiment 1 in the manufacture of a medicament for the treatment of a patient in need an antitumour agent or an antiinflammatory agent or of modulation of lipid or eicosanoid status or function, or of modulation of a lipid metabolising or binding entity activity
13. The method of treatment or use of any one of embodiments 1 to 12 wherein the compound is or comprises a perfluorooctanoic acid, or is more than 75% (for example 80%, 90% or 100%) linear perfluorooctanoic acid or a salt or ester thereof.
14. The method of treatment or use of any one of embodiments 1 to 12, wherein the compound is or comprises a perfluoheptanoic acid or salt or ester thereof.
15. The compound, method or use of any one of embodiments 1 to 12 wherein the compound is a perfluoropentanoic acid or salt or ester thereof.
16. The method or use of any one of embodiments 1 to 15 wherein the patient is human.
17. A screening method for identifying a drug-like compound or lead compound for the development of a drug-like compound in which (1) a mammal is exposed to a compound as defined in embodiment 1 or derivative thereof (2) the plasma insulin, glucose, cholesterol and/or triglyceride level of the mammal is measured, and/or bodyweight of the mammal is measured, and/or lipid or eicosanoid status or function of the mammal is measured.
18. The method of embodiment 17 comprising the step of selecting a compound on exposure to which the plasma insulin, glucose, cholesterol and/or triglyceride level of the mammal is changed or reduced, and/or bodyweight or bodyweight increase of the mammal is changed or reduced.
19. A screening method for identifying a drug-like compound or lead compound for the development of a drug-like compound in which (1) a compound as defined in embodiment 1 or derivative thereof is exposed to a PPAR polypeptide (2) the binding of the compound to the PPAR polypeptide is measured or the change in the activity of the PPAR polypeptide is measured.
20. A screening method for identifying a drug-like compound or lead compound for the development of a drug-like compound in which (1) a compound as defined in embodiment 1 or derivative thereof is exposed to a lipid metabolising or binding entity, for example cycloxygenase (for example cyclooxygenase I or cyclooxygenase II) or phospholipase A (for example phospholipase A2) (2) the binding of the compound to the lipid metabolising or binding entity is measured or the change in the activity of the lipid metabolising or binding entity is measured.
21. A screening method for identifying a drug-like compound or lead compound for the development of a drug-like compound in which (1) a cell is exposed to a compound as defined in embodiment 1 (2) the phenotype (for example differentiation) and/or eicosanoid biosynthesis of the cell is measured.
22. The method of embodiment 21 further comprising the step of selecting a compound on exposure to which the phenotype, for example differentiation, of the cell is changed, and/or eicosanoid biosynthesis of the cell is changed, preferably reduced.
23. The use or method of any one of embodiments 1 to 12 wherein the compound is identified or identifiable by a screening method of any one of embodiments 17 to 22.
24. A compound identified or identifiable by a screening method of any one of embodiments 17 to 22 for use in the manufacture of a composition for use as a food supplement or a food additive.
25. A food product comprising a foodstuff and a compound as defined in claim 1 or a compound identified or identifiable by a screening method of any one of embodiments 17 to 22, wherein the food is not laboratory rodent feed.
26. A kit of parts of screening system comprising (1) a library of compounds each as defined in embodiment 1 and (2) a PPAR polypeptide or polynucleotide encoding a PPAR polypeptide, and/or a test mammal.
27. A kit of parts of screening system comprising (1) a library of compounds each as defined in embodiment 1, and (2) a lipid metabolising or binding entity (for example COXI or COXII or phospholipase A2 or lipoxygenase) or polynucleotide encoding a lipid metabolising or binding entity.
28. Any novel compound, use, kit, system or method as herein disclosed.

## Claims

1. Use of a compound selected from perfluorohexanoic acid, perfluoropentanoic acid, perfluorobutanoic acid or perfluoropropionic acid, or a salt or an ester of any thereof, or perfluorooctane, in the manufacture of a medicament for treating a patient in need of modulation of one or both of plasma insulin and plasma glucose.

2. The use of Claim 1 wherein the patient is in need of reduction of one or both of plasma insulin and plasma glucose.

3. Use of a compound as defined in Claim 1 in the manufacture of a medicament for treating a patient who has diabetes or who is at risk of developing diabetes.

4. The use of any of Claims 1-3 wherein the compound is perfluorooctane.

5. The use of any of Claims 1-4 wherein the patient is human.

6. A compound selected from perfluorohexanoic acid, perfluoropentanoic acid, perfluorobutanoic acid or perfluoropropionic acid, or a salt or an ester of any thereof, or perfluorooctane, for use in treating a patient in need of modulation of one or both of plasma insulin and plasma glucose.

7. The compound of Claim 6 wherein the patient is in need of reduction of one or both of plasma insulin and plasma glucose.

8. A compound as defined in Claim 6 for use in treating a patient who has diabetes or who is at risk of developing diabetes.

9. The compound of any of Claims 6-8 wherein the compound is perfluorooctane.

10. The compound of any of Claims 6-9 wherein the patient is human.
